# EUROPEAN PATENT APPLICATION

(11) **EP 1 913 963 A1**
(43) Date of publication of application: **23.04.2008**
(21) Application number: 06781800.5
(22) Date of filing: 27.07.2006
(51) Int. Cl.: A61M 1/10, A61M 1/12, F04B 51/00, F04D 15/00

(54) **METHOD AND DEVICE FOR MEASURING FLOW RATE AND HEAD OF CENTRIFUGAL PUMP, AND CIRCULATION STATE EVALUATION DEVICE FOR PULSATING CIRCULATION SYSTEM**

(30) Priority: 10.08.2005 JP 2005232501
(71) Applicant: National University Corporation Tokyo Medical and Dental University, Tokyo 113-8510 (JP)
(72) Inventor: HOSHI,Hideo, Osaka 562-0025 (JP); TAKATANI, Setsuo, Chiba-shi, Chiba 2630021 (JP); SHINSHI, Tadahiko, Yokohama-shi, Kanagawa 2260016 (JP); ASAMA, Junnichi, Machida-shi, Tokyo 1940021 (JP)
(74) Representative: Grünecker, Kinkeldey, Stockmair & Schwanhäusser Anwaltssozietät
(86) International application number: PCT/JP2006/314889
(87) International publication number: WO 2007/018044

(57) **Abstract**

In centrifugal pumps 10, 30, 60, 70, and 80 having a rotating centrifugal impeller 14, the flow rate andheadof the pumps are estimated on the basis of transverse force applied to the centrifugal impeller 14 during rotation of the centrifugal impeller 14, and evaluation is alsomade for a circulatory state of the pulsating cardiovascular system. Thereby, it is possible to measure the flow rate and head of the centrifugal pumps and evaluate circulation functions during circulatory assistance by the centrifugal pump.

## Description

### [Technical Field]

The present invention relates to a method for measuring the flow rate and head of a centrifugal pump, the apparatus thereof and an apparatus for evaluating a circulatory state of the pulsating cardiovascular system. In particular, it relates to a method for measuring the flow rate and head of a centrifugal pump favorably usable in evaluating cardiac functions of a mechanical auxiliary circulation in which an artificial heart is used, the apparatus thereof and an apparatus for evaluating a circulatory state of the pulsating cardiovascular system.

### [Background Art]

A mechanical circulatory assistance using, for example, a self-contained or externally-attached auxiliary artificial heart is remarkably effective in treating patients with serious cardiac failure who cannot be treated by drug therapy, and the treatment includes (1) alternative treatment before heart transplantation, (2) treatment for attaining recovery of self cardiac functions and (3) semi-permanent usage. The mechanical auxiliary circulation is effective in improving systemic symptoms of patients, and it has been reported that with some patients withdrawn from the auxiliary artificial heart as described in the above treatment (2). In Japan where heart transplantation has not become widespread, the treatment (2) is remarkably effective and regarded as promising. Further, in view of combination with regenerative medicine, this treatment has great therapeutic possibilities. Some of the present inventors have proposed a self-contained or externally-attached continuous-flow disposable magnetic levitation centrifugal blood pump favorably feasible in providing the above-described mechanical circulatory assistance as in Japanese Published Unexamined Patent Application No. 2005-118237 (hereinafter, referred to as Patent Document 1) and "Complete Non-Contact Type Rotary Centrifugal Blood Pump Using Magnetic Bearing" authored by Setsuo Takaya, annual report of the Institute of Biomaterials and Bioengineering, Tokyo Medical and Dental University, Vol. 38 (2004) pages 38 to 41 (hereinafter, referred to as Non-patent Document 1).

Further, as a detection system and a motor speed control system for avoiding sucking phenomena in providing the circulatory assistance in the above type of steady flow pump, proposed are (1) those in which motor current waveform is used, (2) those in which a blood flow meter attached to a blood transmitting tube is used, and (3) those in which the flow rate is estimated by referring to the rotation number of a motor and a pressure sensor attached inside a pump.

These systems are effective in avoiding sucking phenomena found in auxiliary circulation, however they are not feasible in evaluating cardiac functions during auxiliary circulation.

It may be considered an idea that the frequency of motor current waveform is analyzed and a power spectrum is used to evaluate cardiac functions on the basis of motor current. This idea has problems such as the necessity of previous calibration for individuals, necessity of monitoring and accumulating data over time, difficulty in performing continuous monitoring due to necessity of mathematical calculation processing and a greater influence of noises from motor current on the output result.

Further, there is another problem that a flow meter or the like, which is a separate device, is needed in order to measure a pump output amount.

### [Disclosure of the Invention]

The present invention has been made for solving the above-described conventional problems, a first object of which is to calculate the output amount of a centrifugal pump without using a flow meter or the like.

A second object of the present invention is to evaluate a reliable circulatory state which is continuous, highly stable and able to make a highly sensitive evaluated output.

The invention according to claim 1 is that in which in a centrifugal pump having a rotating centrifugal impeller, the flow rate of the pump is estimated on the basis of transverse force applied to the centrifugal impeller during rotation of the centrifugal impeller, thereby attaining the first object.

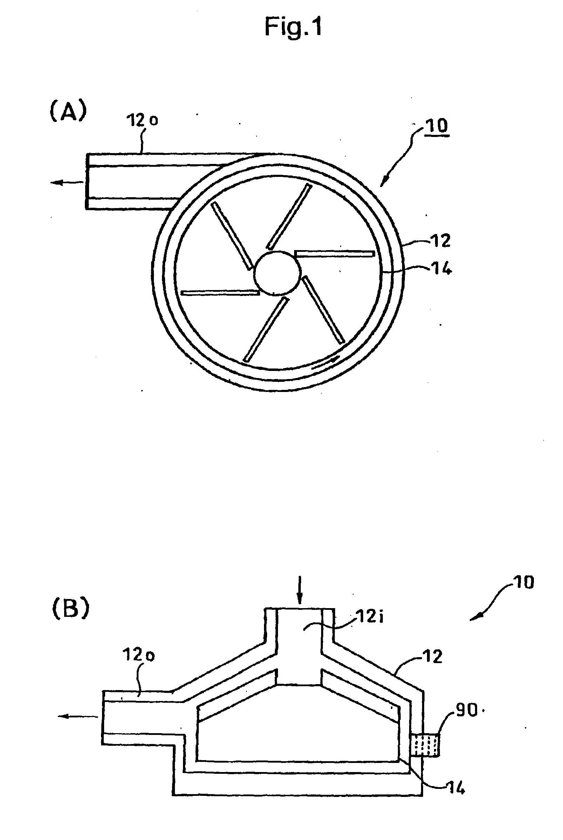

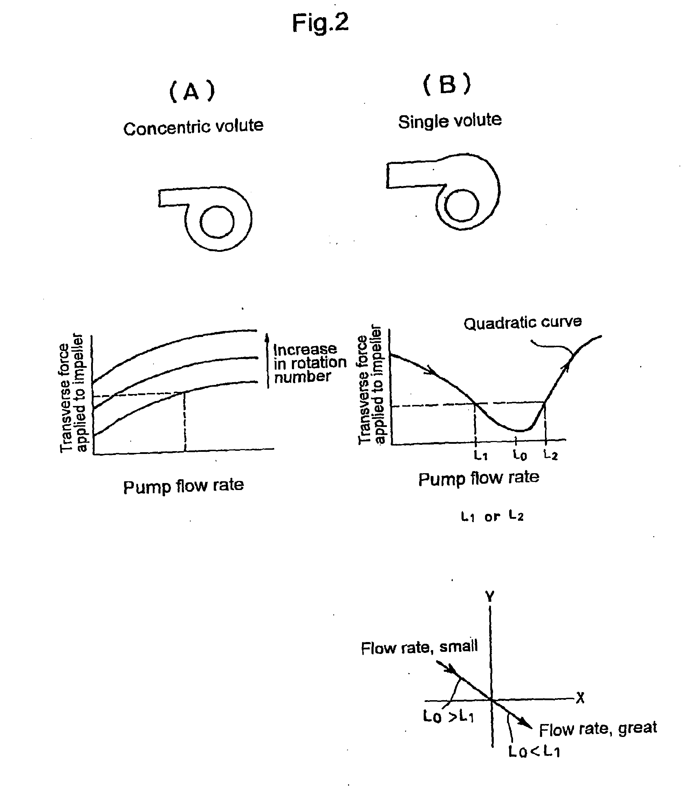

In other words, when a centrifugal pump 10 as exemplified in Fig. 1 outputs steadily from an outlet 120 a fluid flowing from an inlet 12i at a constant rotation number of a centrifugal impeller (also referred to as impeller) 14, transverse force is developed on the centrifugal impeller 14 due to a change in the fluid force inside a pump casing 12. Theoretically, the transverse force is decided mainly by the shape of a volute and caused by imbalance of the fluid force inside the casing. In a concentric volute given above in Fig. 2 (A) and a single volute given above in Fig. 2(B), the force is applied from the center toward the outlet.

The relationship of the pump flow rate and rotation number of the concentric volute with transverse force applied to the impeller is shown in the middle of Fig. 2 (A), and the relationship of the pump flow rate of the single volute with transverse force applied to the impeller is shown in the middle of Fig. 2 (B). As apparent from Fig. 2, both of the concentric volute and single volute can be calculated for the flow rate from the relationship of the rotation number with transverse force applied to the impeller by measuring transverse force applied to the impeller or pressure inside the pump casing which is responsible for developing the transverse force.

For this purpose, preferable is such a concentric volute that has a linear relationship of the transverse force with the flow rate shown in the middle of Fig. 2 (A).

It is noted that, as shown in the middle of Fig. 2 (B), the single volute gives a quadratic curve having an extreme value in the vicinity of a maximum efficiency of the pump. Therefore, calculation of transverse force applied to the impeller results in two flow rates, L1 and L2, which can be estimated. However, as shown below in Fig. 2 (B), the flow rate can be estimated by determining whether the flow rate concerned is located right or left from an extreme value L0.

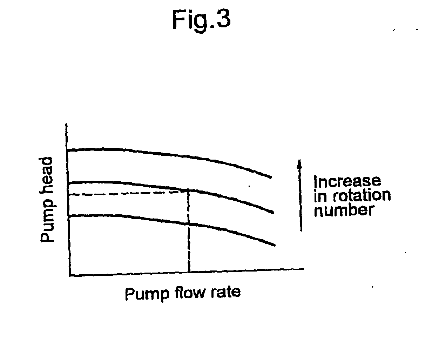

It is also possible to calculate the pump head on the basis of the flow rate of the pump and rotation number of the centrifugal impeller by referring to the pressure-flow rate diagram given in Fig. 3.

Therefore, centrifugal impellers supported with certain spring rigidity such as a magnetic bearing and a hydrodynamic bearing can be measured for the displacement to estimate the transverse force; flow rate and head thereof. Further, contact bearings such as a shaft seal type and a pivot type can also be estimated for the flow rate and head.

It is noted that a double volute is effective in mitigating or reducing an imbalanced fluid force inside a casing and not appropriately used on development of a transverse force.

The invention according to claim 2 is that in which transverse force applied to the centrifugal impeller is detected by referring to displacement behavior of the centrifugal impeller.

The invention according to claim 3 is that in which transverse force applied to the centrifugal impeller is detected by referring to a control value for retaining the centrifugal impeller at a predetermined position.

The invention according to claim 4 is that in which transverse force applied to the centrifugal impeller is detected by referring to the pressure inside a pump casing responsible for developing the transverse force.

The invention according to claim 5 is that in which a pump head is estimated on the basis of the flow rate of a pump estimated as described above.

The invention according to claim 6 is to provide an apparatus for measuring the flow rate of a centrifugal pump having a rotating centrifugal impeller, the apparatus having means for detecting transverse force applied to the centrifugal impeller during rotation of the centrifugal impeller and means for estimating the flow rate of the pump on the basis of the transverse force.

The invention according to claim 7 is that in which the means for detecting the transverse force is a displacement sensor for detecting the displacement of the centrifugal impeller.

The invention according to claim 8 is that in which the means for detecting the transverse force is means for detecting a control value for retaining the centrifugal impeller at a predetermined position.

The invention according to claim 9 is that in which the means for detecting the transverse force is a pressure sensor for detecting the pressure inside a pump casing responsible for developing the transverse force.

The invention according to claim 10 is to provide an apparatus for measuring the head of a centrifugal pump in which the pump is estimated for the head on the basis of the flow rate of the pump as estimated above.

The invention according to claim 11 is an apparatus for evaluating a circulatory state of the pulsating cardiovascular system assisted in circulation by using a centrifugal pump having a rotating centrifugal impeller, which is provided with means for detecting transverse force applied to the centrifugal impeller during rotation of the centrifugal impeller and means for evaluating a circulatory state of the pulsating cardiovascular system on the basis of the transverse force, thereby attaining the second object.

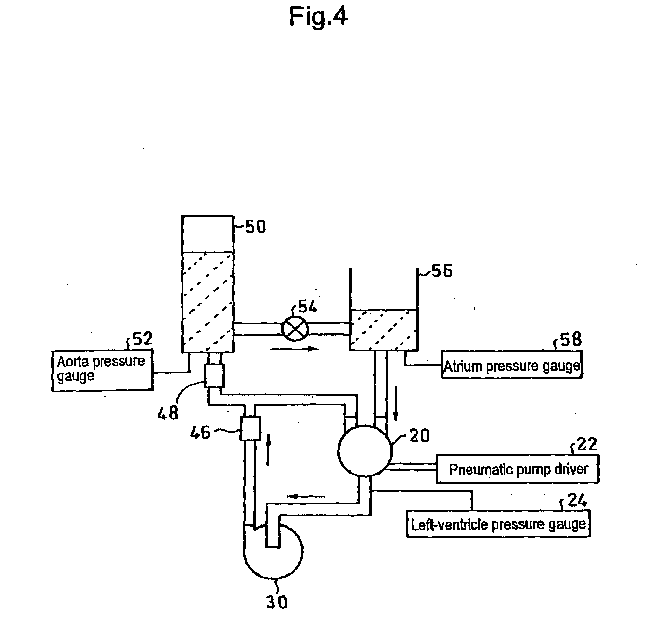

Specifically, the centrifugal impeller supported in anon-contact manner on auxiliary circulation is subjected to micro-vibration in a range between 0 µm to 20 µm by a pulsating flow component of the left ventricle and a fluid force inside the pump. The inventor and others have evaluated the phenomenon by a mock circulation circuit shown in Fig. 4, thereby clarifying the relationship of the behavior models (pump theory andmagneticbearing theory) with cardiac functions.

In Fig. 4, reference numeral 20 is amockheart driven, for example, by a pneumatic pump driver 22; 30, a magnetic levitation centrifugal blood pump proposed, for example, by some of the inventors in Patent Document 1 and Non-patent Document 1; 50, a compliance tank on the aorta; 54, resistance corresponding to peripheral resistance in the body; 56, a reservoir on the atrium; 24, a left-ventricle pressure gauge; 46, a pump flow meter; 48, an aorta flow meter; 52, an aorta pressure gauge; and 58, an atrium pressure gauge.

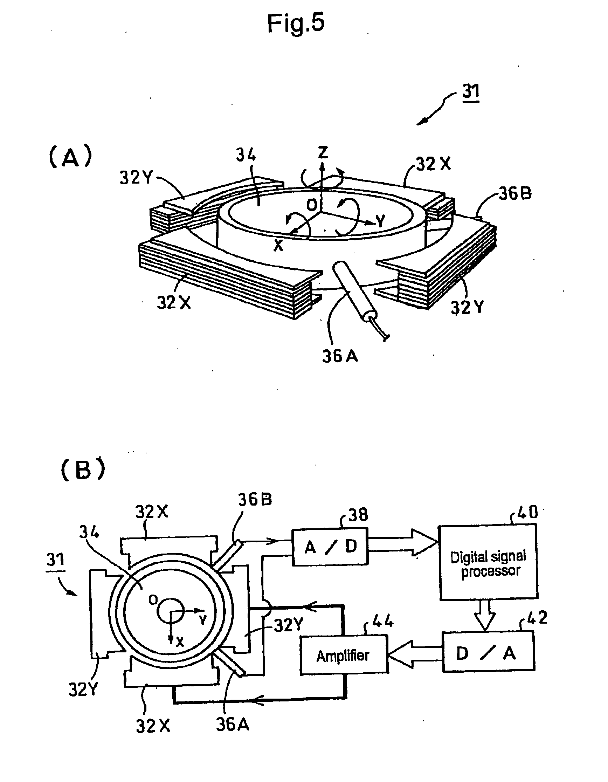

As shown in detail in Fig. 5, the centrifugal blood pump 30 is provided with a rotor 34 magnetically levitated by electromagnets 32X, 32Y respectively in X direction and Y direction, displacement sensors 36A, 36B, for example, made up of eddy current sensors for detecting the displacement of the rotor 34 in two directions, an A/D converter 38 for converting analog signals output from the displacement sensors 36A, 36B to digital signals, a digital signal processor 40 for processing the output of the A/D converter 38 to output a signal for feedback controlling the position of the rotor 34, a D/A converter 42 for converting the output of the digital signal processor 40 to analog signals for giving it to the electromagnets 32X, 32Y, and a magnetic bearing 31 equipped with an amplifier 44 for amplifying the output of the D/A converter 42 to input it into the electromagnets 32X, 32Y, in which the centrifugal impeller (not illustrated) is molded on the rotor 34 and also a driving magnet (not illustrated) for rotating and driving the centrifugal impeller in a non-contact manner is embedded into the rotor 34 (refer to Non-patent Document 1).

In the above-described mock circulation circuit, behavior of the centrifugal impeller (gap with the pump casing) in a steady flow state at which a mock heart 20 is halted is as shown in Fig. 6(A). However, it has been found that when the mock heart 20 is driven to give pulsation, the behavior is as shown in Fig. 6(B), and the pulsation number can be detected by referring to the period P, as shown in Fig. 7, and the pulse pressure, left ventricle pressure and pump flow rate can be detected by referring to the amplitude A. Fig. 8 shows the relationship between the maximum pressure of the left ventricle and the amplitude A of the impeller. The present invention has been made on the basis of these findings.

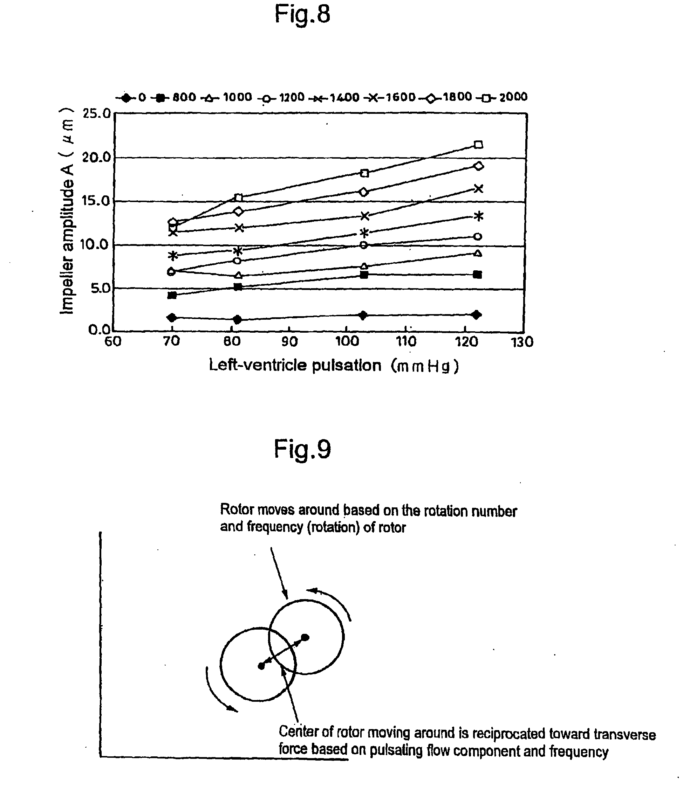

It is desirable that two or more pulsating components are detected with respect to X and Y directions. This is because the rotor rotates in a complicated manner under the pulsating flow and, as shown in Fig. 9, the rotor moves around according to the frequency of the rotation number of the rotor and the rotational center is reciprocated toward the transverse force by a pulsating flow component according to the frequency of the pulsating flow component.

According to the present invention, an output amount of the centrifugal pump can be measured without using a flow meter or the like.

Further, where evaluation is made for a circulatory state of the pulsating cardiovascular system according to the present invention, it is possible to evaluate not only an auxiliary flow rate during auxiliary circulation but also cardiac functions of a patient's own heart. As a result, it is possible to evaluate a reliable circulatory state which is continuous, highly stable and able to make a highly sensitive evaluated output, as compared with a method in which motor current waveform signals are used for evaluation. Further, since, for example, variation in ventricular pressure gives a direct influence on behavior of the centrifugal impeller, it is possible to obtain a remarkably reliable output.

Therefore, the present invention is able to evaluate cardiac functions and an auxiliary circulatory state conveniently and continuously on a steady basis, eliminating the necessity of a catheter, diagnostic image apparatus or heart straining examinations using medication in the treatment of cardiac failure during auxiliary circulation. For this reason, it is effective for rehabilitation of patients with cardiac failure and recovery of cardiac functions. Further, the rotation number of a centrifugal impeller is changed on the basis of the output results obtained from the apparatus of the present invention, by which the output flow rate of the pump can be adjusted to a target flow rate. Still further, it is possible to instantly detect abnormal phenomena such as sucking, backflow and kinking during the auxiliary circulation.

### [Brief Description of the Drawings]

Fig. 1 covers a plan view (A) and a sectional view (B) showing the constitution of an example of the centrifugal pump, which is a target of the present invention.
Fig. 2 is a diagram illustrating characteristics of transverse force applied to an impeller in a concentric volute, which shows a principle of measuring a pump flow rate according to the present invention.
Fig. 3 is also a diagram illustrating characteristics of pressure/flow rate in the centrifugal pump.
Fig. 4 is a circuit diagram illustrating one example of a mock circulation circuit used in evaluating a circulatory state of the present invention.
Fig. 5 covers a perspective view (A) and a circuit diagram (B) illustrating a magnetic bearing used in the centrifugal blood pump.

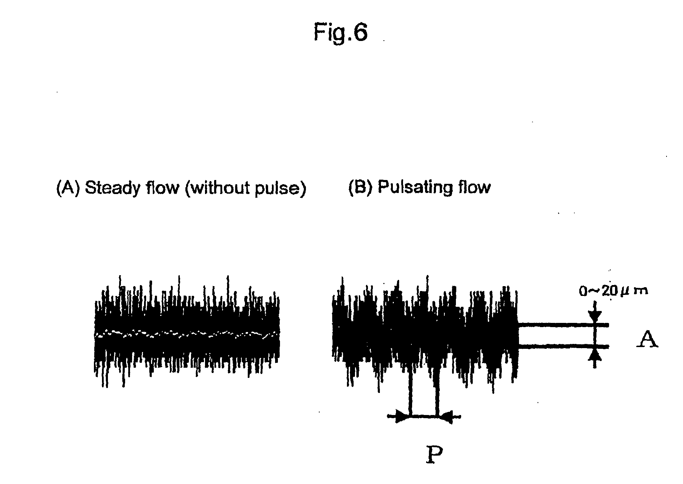

Fig. 6 is a drawing comparatively showing the change in behavior due to the presence or absence of pulsation of a magnetic levitation centrifugal impeller, which explains a principle of evaluating a circulatory state in the present invention.

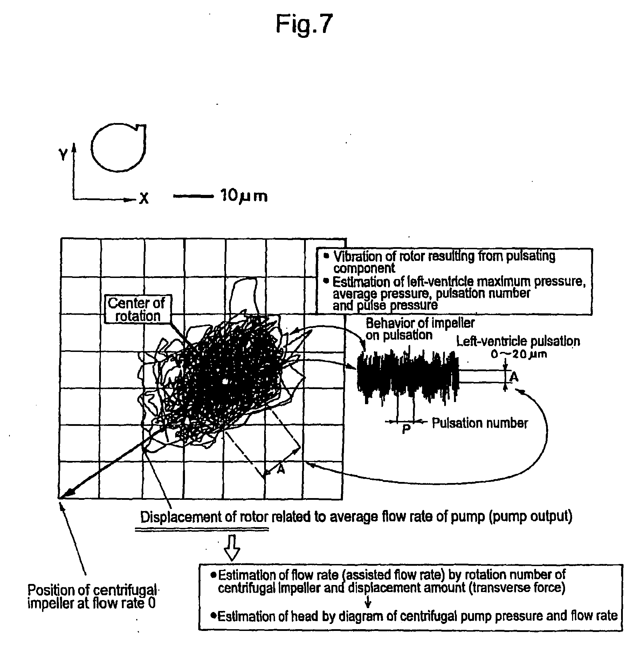

Fig. 7 is also a diagram showing a method for estimating behavior of the centrifugal impeller, heart rate, auxiliary flow rate and pump head when pulsation is present.
Fig. 8 is also a diagram showing the relationship between the maximum pressure of the left ventricle and the impeller amplitude.
Fig. 9 is also a diagram showing a detailed behavior of a rotor.

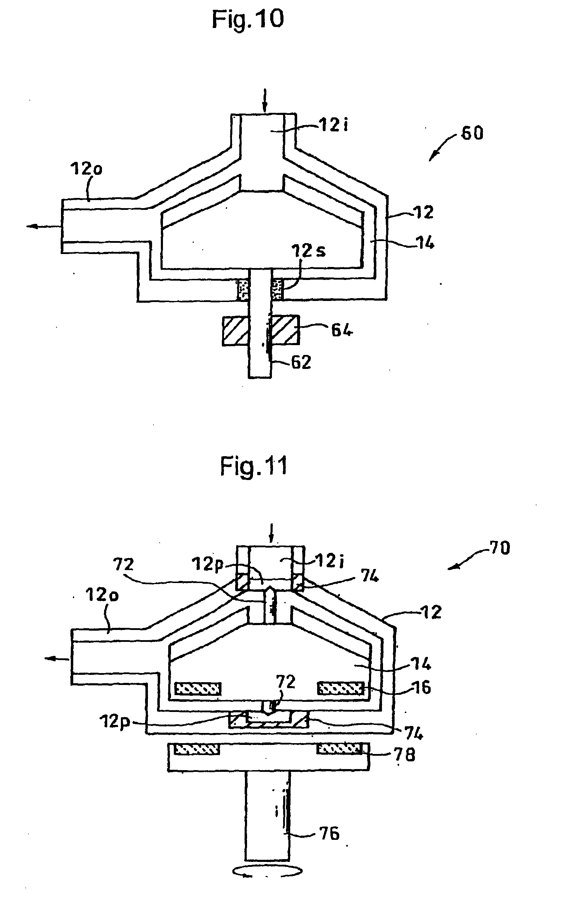

Fig. 10 is a sectional view showing Embodiment 1 for measuring the flow rate of the centri fugal pump according to the present invention.
Fig. 11 is also a sectional view showing Embodiment 2.

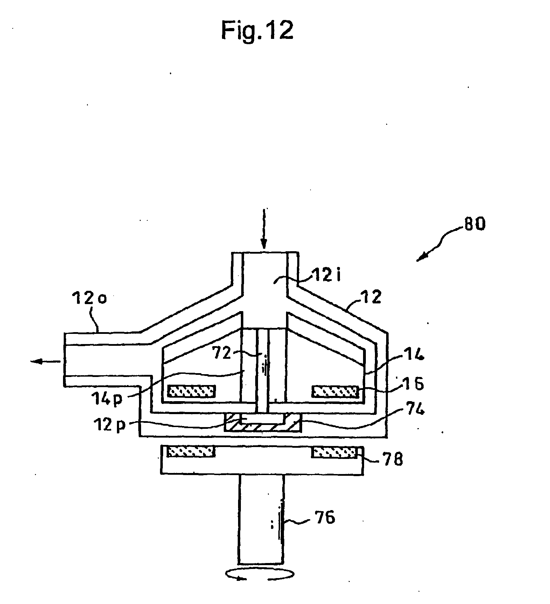

Fig. 12 is also a sectional view showing Embodiment 3.

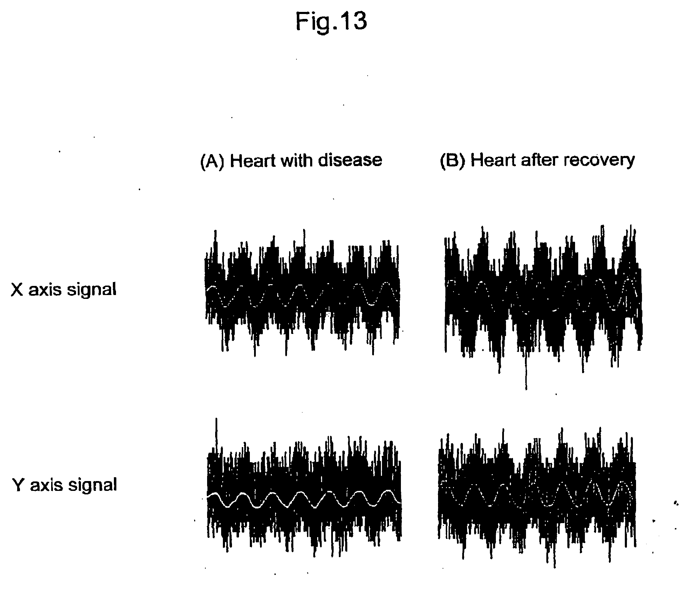

Fig. 13 is a drawing showing one example of measurement results in the present invention.

### [Best Mode for Carrying Out the Invention]

Hereinafter, an explanation will be made for embodiments of the present invention by referring to the drawings.

Embodiment 1 of the present invention is that in which the present invention is applied for measuring the flow rate of a shaft seal contact bearing equipped centrifugal pump which is directly connected to the shaft. As shown in Fig. 10, in a centrifugal pump 60 provided with a centrifugal impeller 14 rotated by a shaft 62 inserted into a shaft seal 12s of the pump casing 12, a load cell 64 is installed on the shaft 62, thereby detecting transverse force applied to a centrifugal impeller 14.

Embodiment 2 of the present invention is that in which the present invention is applied for measuring the flow rate of a magnetic-coupling sealless centrifugal pump vertically equipped with a vertical pivot-type contact bearing. As shown in Fig. 11, in a centrifugal pump 70 provided with pivot brackets 12p vertically arranged inside a pump casing 12 and a centrifugal impeller 14 supported by a vertically-extending pivot shaft 72, a load cell 74 is respectively installed on the vertically-arranged pivot brackets 12p, thereby detecting transverse force applied to the centrifugal impeller 14 with reference to a sum of the outputs. This pump is arranged as described above because there is found uneven contact, the effect of which is to be avoided.

In the drawing, reference numeral 16 is a permanent magnet on the driven side which is embedded into the centrifugal impeller 14; 76, a shaft; and 78, a permanent magnet on the driving side which is embedded into the shaft 76.

Embodiment 3 of the present invention is that in which the present invention is applied for measuring the flow rate of a magnetic coupling sealless centrifugal pump equipped with a pivot-type contact bearing only on the lower side. As shown in Fig. 12, in a centrifugal pump 80 provided with a pivot bracket 12p disposed inside the pump casing 12 only on the lower side and a centrifugal impeller 14 supported by a pivot shaft 72 extending below, a load cell 74 is installed on the pivot bracket 12p, thereby detecting transverse force applied to the centrifugal impeller 14. In this pump, there is no fear of uneven contact and one load cell will be enough.

In the drawing, reference numeral 14p is a pivot bracket of the centrifugal impeller 14.

It is noted that in place of the pivot bracket, a journal or a thrust slide bearing can be used.

According to Embodiments 1 to 3, since the pumps are provided with a contact bearing, they are able to directly detect transverse force.

Embodiment 4 of the present invention is that in which the present invention is applied to a centrifugal pump provided with a magnetic bearing 31 given in Fig. 5, thereby detecting the displacement of a centrifugal impeller with reference to the outputs of the displacement sensors 36A and 36B.

Specifically, in the present embodiment, since control is taken so that a minimal steady control current value can be given as a target value in view of reducing the electric power consumption, transverse force can be measured by referring to the behavior of an impeller, although the target value varies.

It is noted that where a position to be levitated is fixed in advance to a predetermined position and feedback control is taken to give a target value to the position, the transverse force may be detected by referring to the feedback control value.

Embodiment 5 of the present invention is such that, as shown in Fig. 1, a pressure sensor 90 is installed at a site where the pressure varies to a great extent, for example, in the vicinity of the outlet of a pump casing 12, thereby detecting the pressure inside the pump casing 12.

Fig. 13 comparatively shows examples of signals in X axis and Y axis directions for a heart having cardiac disease (A) and the heart after recovery (B), from which the recovery of cardiac functions is obvious at a glance.

### [Industrial Applicability]

It is noted that in the previously described embodiments, the present inventionhas been applied to mechanical circulatory assistance systems. However, the present invention shall not be limited in application thereto and is also applicable not only for evaluating a circulatory state of cardiovascular systems other than those for humans but also for measuring the flow rate of a centrifugal pump alone.

Further, the type of non-contact type bearing is not limited to a magnetic bearing based on magnetic levitation but may include a hydrodynamic bearing based on dynamic levitation and others.

## Claims

1. A method for measuring the flow rate of a centrifugal pump, wherein the centrifugal pump is provided with a rotating centrifugal impeller, the flow rate of the pump is estimated on the basis of transverse force applied to the centrifugal impeller during rotation of the centrifugal impeller.

2. The method for measuring the flow rate of a centrifugal pump as set forth in claim 1, wherein transverse force applied to the centrifugal impeller is detected by referring to displacement behavior of the centrifugal impeller.

3. The method for measuring the flow rate of a centrifugal pump as set forth in claim 1, wherein transverse force applied to the centrifugal impeller is detected by referring to a control value for retaining the centrifugal impeller at a predetermined position.

4. The method for measuring the flow rate of a centrifugal pump as set forth in claim 1, wherein transverse force applied to the centrifugal impeller is detected by referring to the pressure inside a pump casing responsible for developing the transverse force.

5. A method for measuring the head of a centrifugal pump, wherein the head of the pump is estimated on the basis of the flow rate of the pump estimated in any one of claim 1 to claim 4.

6. An apparatus for measuring the flow rate of a centrifugal pump comprising a rotating centrifugal impeller, which is provided with
means for detecting transverse force applied to the centrifugal impeller during rotation of the centrifugal impeller
and means for estimating the flow rate of the pump on the basis of the transverse force.

7. The apparatus for measuring the flow of a centrifugal pump as set forth in claim 6, wherein the means for detecting the transverse force is a displacement sensor for detecting the displacement of the centrifugal impeller.

8. The method for measuring the flow rate of a centrifugal pump as set forth in claim 6, wherein the means for detecting the transverse force is means for detecting a control value for retaining the centrifugal impeller at a predetermined position.

9. The apparatus for measuring the flow rate of a centrifugal pump as set forth in claim 6, wherein the means for detecting the transverse force is a pressure sensor for detecting the pressure inside a pump casing responsible for developing the transverse force.

10. An apparatus for measuring the headof a centrifugal pump, wherein the head of the pump is estimated on the basis of the flow rate of the pump estimated in any one of claim 6 to claim 9.

11. An apparatus for evaluating a circulatory state of the pulsating cardiovascular system assisted in circulation by using a centrifugal pump having a rotating centrifugal impeller, which is provided with
means for detecting transverse force applied to the centrifugal impeller during rotation of the centrifugal impeller
and means for evaluating a circulatory state of the pulsating cardiovascular system on the basis of the transverse force.
